# EUROPÄISCHE PATENTANMELDUNG

(11) **EP 3 632 475 A1**
(43) Veröffentlichungstag der Anmeldung: **08.04.2020**
(21) Anmeldenummer: 18206220.8
(22) Anmeldetag: 14.11.2018
(51) Int. Cl.: A61L 2/10, C02F 1/32

(54) **ANORDNUNG FÜR EINE VORRICHTUNG ZUM DESINFIZIEREN EINES FLUIDS UND VORRICHTUNG**

(30) Priorität: 04.10.2018 DE 102018124504
(71) Anmelder: Hytecon AG, 6006 Luzern (CH)
(72) Erfinder: KLINK, Maximilian, 6006 Luzern (CH)
(74) Vertreter: Bittner, Thomas L.

(57) **Zusammenfassung**

Die Erfindung betrifft eine Anordnung für eine Vorrichtung zum Desinfizieren eines Fluids mit einem Behälter (1); einem Reaktorraum (2), welcher in dem Behälter (1) angeordnet und eingerichtet ist, ein zu desinfizierendes Fluid aufzunehmen, das über einen Einlass des Reaktorraums (2) in diesen gelangen und über einen Auslass des Reaktorraums (2) diesen verlassen kann; und einer UV-Bestrahlungseinrichtung (9), die lichtemittierende UV-Dioden (6) aufweist, mit denen UV-Lichtstrahlen zum Desinfizieren des Fluids in den Reaktorraum (2) einstrahlbar sind; wobei der Reaktorraum (2) in dem Behälter (1) mit einer Durchflussstrecke (3) in einem von einer Behälterwandung (5) umgebenen Durchflussrohr (4) gebildet ist und die lichtemittierenden UV-Dioden (6) an der Behälterwandung (5) im Bereich des Durchflussrohres (4) entlang der Durchflussstrecke (3) verteilt angeordnet sind, um die UV-Lichtstrahlen auf die Durchflussstrecke (3) einzustrahlen. Weiterhin ist eine Vorrichtung zum Desinfizieren eines Fluids vorgesehen.

## Beschreibung

Die Erfindung betrifft eine Anordnung für eine Vorrichtung zum Desinfizieren eines Fluids sowie eine Vorrichtung.

### Hintergrund

Das Desinfizieren von Fluiden, beispielsweise Wasser oder Trinkwasser, ist in Verbindung mit verschiedensten Anwendungen vorgesehen. Ein verschmutztes oder nicht geeignetes Fluid wird desinfiziert, um dann in desinfizierter Form für eine weitere Verwendung bereitgestellt zu werden. Beispielsweise wird auf diese Weise Wasser desinfiziert, um es zum Beispiel als Trinkwasser bereitzustellen, insbesondere an einer Verbrauchsstelle ("Point of Use").

Im Dokument WO 2018 / 050144 A1 ist eine dezentrale Wasserdesinfektionsvorrichtung offenbart. UV-Lichtstrahlen von UV-lichtemittierenden Dioden werden genutzt, um das Wasser in einem Reaktorraum zu desinfizieren. Im Dokument DE 10 2016 122 075 A1 sind ein Arbeitsverfahren und eine Vorrichtung zur Überwachung einer UV-Desinfektionsanlage offenbart. UV-Lichtstrahlen werden genutzt, um ein Fluid in einem Reaktorraum zu desinfizieren.

### Zusammenfassung

Aufgabe der Erfindung ist es, eine Anordnung für eine Vorrichtung zum Desinfizieren eines Fluids sowie eine Vorrichtung zum Desinfizieren eines Fluids anzugeben, mit denen eine verbesserte Desinfektion des Fluids erreichbar ist.

Zur Lösung ist eine Anordnung für eine Vorrichtung zum Desinfizieren eines Fluids nach dem unabhängigen Anspruch 1 geschaffen. Weiterhin ist eine Vorrichtung zum Desinfizieren eines Fluids nach dem nebengeordneten Anspruch 11 vorgesehen. Ausgestaltungen sind Gegenstand von abhängigen Unteransprüchen.

Nach einem Aspekt ist eine Anordnung für eine Vorrichtung zum Desinfizieren eines Fluids geschaffen, die einen Behälter sowie einen Reaktorraum aufweist, welcher in dem Behälter angeordnet ist. Der Reaktorraum ist eingerichtet, ein zu desinfizierendes Fluid aufzunehmen, das über einen Einlass des Reaktorraums in diesen gelangen und über einen Auslass des Reaktorraums diesen verlassen kann. Weiterhin ist eine UV-Bestrahlungseinrichtung vorgesehen, die lichtemittierende UV-Dioden aufweist, mit denen UV-Lichtstrahlen zum Desinfizieren des Fluids in den Reaktorraum einstrahlbar sind. Der Reaktorraum ist in dem Behälter mit einer Durchflussstrecke in einem von einer Behälterwandung umgebenen Durchflussrohr gebildet. Die lichtemittierenden UV-Dioden sind an der Behälterwandung im Bereich des Durchflussrohres entlang der Durchflussstrecke verteilt angeordnet, um die UV-Lichtstrahlen auf die Durchflussstrecke einzustrahlen.

Weiterhin ist eine Vorrichtung zum Desinfizieren eines Fluids, mit einer solchen Anordnung vorgesehen.

Mit der Anordnung kann ein Reaktor für eine Vorrichtung zum Desinfizieren eines Fluids bereitgestellt sein, beispielsweise zum Desinfizieren von Wasser, insbesondere Trinkwasser. Wegen der Ausbildung des Reaktorraums mit der Durchflussstrecke in dem Durchflussrohr kann der Reaktor auch als Rohrreaktor bezeichnet werden.

Die Anordnung der lichtemittierenden UV-Dioden kann sich über die gesamte Länge der Durchflussstrecke an der Behälterwandung entlang erstrecken.

Der Einlass des Reaktorraums und / oder der Auslass des Reaktorraums können im Bereich von Endabschnitten des Durchflussrohres ausgebildet sein.

In einem Beispiel können auf der inneren Oberfläche der Behälterwandung im Bereich des Durchflussrohres strömungsleitende Elemente angeordnet sein, um das zu desinfizierende Fluid beim Durchfließen oder Durchströmen des Durchflussrohres zu leiten oder zu führen, beispielsweise zum Induzieren von Wirbeln, was die Bestrahlung mit dem UV-Licht verbessern kann. Auch eine spiralförmige Strömung des Fluids in dem Durchflussrohr kann wahlweise mit Hilfe der strömungsleitenden Elemente induziert werden.

Zumindest ein Teil der lichtemittierenden UV-Dioden kann mindestens eine Linienanordnung von Dioden ausbildend an der Behälterwandung angeordnet sein. Entlang der Linienanordnung von Dioden oder linienförmigen Diodenanordnung können die lichtemittierenden UV-Dioden mit gleichen oder unterschiedlichen Abständen zwischen benachbarten Dioden angeordnet sein. Entlang der Linienanordnung von Dioden kann mittels der UV-Dioden eine Kette von UV-Strahlungsquellen gebildet sein. Die Linienanordnung von Dioden kann einen oder mehrere Abschnitte der folgenden Art aufweisen: gerade Linie, Zickzack-Linie, spiralförmige Linie und Wellenlinie. Eine oder mehrere der Linienanordnungen von Dioden können sich im Wesentlichen parallel zur Durchgangsrichtung des Durchflussrohres erstrecken. Weist die Behälterwandung innenseitig strömungsleitende Elemente auf, können eine oder mehrere Linienanordnungen von Dioden entlang der strömungsleitenden Elemente verlaufen, zum Beispiel parallel hierzu. Alternativ oder ergänzend zu den Linienanordnungen von Dioden können lichtemittierende UV-Dioden einzeln oder in Gruppen im Bereich der Behälterwandung an der Durchflussstrecke angeordnet sein.

Die Linienanordnung von Dioden kann sich entlang einer Schlitzausnehmung der Behälterwand erstrecken. Bei dieser oder anderen Ausführungsformen können die lichtemittierenden UV-Dioden in einem zugeordneten Durchbruch der Behälterwandung eingelassen sein, wobei die lichtemittierende UV-Dioden gedichtet in dem Durchbruch angeordnet sind, so dass die Durchflussstrecke fluiddicht gegenüber der Umgebung des Behälters abgedichtet ist. Es kann vorgesehen sein, dass die lichtemittierende UV-Dioden hinter einem für die UV-Lichtstrahlen transparenten Fenster angeordnet sind, welches gedichtet in die Behälterwandung integriert ist.

Bei den verschiedenen Ausführungsformen ist ein Abstrahlbereich der lichtemittierenden UV-Dioden, in welchen das von der Diode erzeugte UV-Licht abgestrahlt wird, zu der Durchflussstrecke für das zu desinfizierende Fluid hin ausgerichtet.

Die lichtemittierenden UV-Dioden können an der Behälterwandung im Bereich des Durchflussrohres um die Durchflussstrecke herum verteilt angeordnet sein. Um die Durchflussstrecke herum können mehrere Linienanordnungen von Dioden ausgebildet sein, zum Beispiel mehrere gerade Linienanordnungen von Dioden, die sich in Durchgangsrichtung des Durchflussrohres erstrecken. Eine Anordnung von paarweise einander gegenüberliegenden Linienanordnungen kann vorgesehen sein.

Den lichtemittierenden UV-Dioden können jeweils eine außerhalb des Reaktorraums angeordnete Kühleinrichtung zugeordnet sein, welche eingerichtet ist, die lichtemittierenden UV-Dioden im Betrieb zu Kühlen. Die Kühleinrichtung dient dazu, von der lichtemittierenden UV-Diode im Betrieb erzeugte Wärmeenergie abzuführen, um so einen sicheren Betrieb der lichtemittierenden UV-Dioden zu unterstützen. Eine Aktive Kühlung, bei der die Wärmeenergie mittels eines Kühlmittels abgeführt wird, und i oder eine passive Kühlung können vorgesehen sein, welche Kühlmittelfrei ist und zum Beispiel Kühlkörper aufweist.

Die Kühleinrichtung kann sich vom Behälter weg erstreckenden Kühlelementen aufweisen. Die Kühlelemente können beispielsweise Kühlrippen aufweisen, die sich vom Behälter weg erstrecken, um so die im Betrieb erzeugte Wärmeenergie abzuleiten. Auch aktive Kühlelemente sich vom Behälter weh erstreckend ausgeführt sein.

Die Kühleinrichtung kann einen Kühlmitteldurchgang aufweisen, welcher im Betrieb zum Kühlen von einen Kühlmittel durchströmbar ist, um eine aktive Kühlung bereitzustellen. Der Kühlmitteldurchgang kann Kanäle und / oder Hohlräume umfassen, um im Betrieb ein Kühlmittel aufzunehmen, welches zur Ableitung der von den lichtemittierenden UV-Dioden erzeugten Wärmeenergie dient. Hierbei kann ein geschlossener Kühlmittelkreislauf vorgesehen sein, bei dem aus einem Kühlmittelreservoir Kühlmittel in die Kühlmitteldurchgänge zugeführt und dann wieder aus diesen zum Reservoir abgeführt wird.

Kühleinrichtungen können für wenigstens zwei der lichtemittierenden UV-Dioden als gemeinsame Kühleinrichtung ausgebildet sein. Zum Beispiel können benachbart zueinander angeordnet lichtemittierende UV-Dioden eine gemeinsame Kühleinrichtung aufweisen, beispielsweise in der Linienanordnung von Dioden. Hierbei kann pro Linienanordnung von Dioden eine einzige Kühleinrichtung für alle hiervon umfassten lichtemittierenden UV-Dioden vorgesehen sein. Die gemeinsame Kühleinrichtung kann gemeinsame Kühlrippen oder -körper und / oder gemeinsame Kühlmitteldurchgänge für die lichtemittierenden UV-Dioden umfassen.

Die Behälterwandung kann wenigstens oberflächenseitig zumindest abschnittweise aus einem die UV-Lichtstrahlen diffus reflektierenden Material sein. Das Ausbilden der Behälterwandung oberflächenseitig aus dem die UV-Lichtstrahlen diffus reflektierenden Material kann mit Hilfe eines Vollmaterialabschnitts der Behälterwand oder mittels einer oberflächenseitigen Beschichtung auf der Behälterwand bereitgestellt sein.

Die Behälterwandung kann insbesondere zwischen den Linienanordnungen von Dioden wenigstens oberflächenseitig abschnittweise oder vollständig aus dem die UV-Lichtstrahlen diffus reflektierenden Material sein.

Es kann vorgesehen sein, dass den lichtemittierenden UV-Dioden gegenüberliegend ein Abschnitt der Behälterwandung angeordnet ist, welcher wenigstens oberflächenseitig abschnittweise oder vollständig aus dem die UV-Lichtstrahlen diffus reflektierenden Material ist, so dass das von den lichtemittierenden UV-Dioden emittierte Licht auf diesen Abschnitt, welcher vollständig frei von UV-Dioden sein kann, einfällt und diffus reflektiert wird.

Im Fall der Beschichtung kann diese eine Mindestdicke von etwa einem Millimeter aufweisen, um Verluste von UV-Lichtstrahlen im Bereich der Oberfläche zu mindern. Der Reaktorraum kann im Bereich der Durchflussstrecke wenigstens oberflächenseitig vollständig aus dem die UV-Lichtstrahlen diffus reflektierenden Material sein. Als Material für die Behälterwandung kann in den verschiedenen Ausführungsformen zum Beispiel Quarz oder Polytetrafluorethylen zum Einsatz kommen. Polytetrafluorethylen kann auch genutzt werden, um eine Oberflächenbeschichtung bereitzustellen.

Hierdurch ist erreicht, dass die zur Desinfektion des Fluids eingestrahlten Lichtstrahlen in möglichst großem Umfang von der Schicht reflektiert und in das zu desinfizierende Fluid, beispielsweise Wasser, zurückgeworfen werden, um so wahlweise mehrfach zum Desinfizieren beizutragen. Es wurde festgestellt, dass bei der vorgesehenen Schichtdicke von wenigstens 1mm für die Beschichtung oder das Vollmaterial ein Verlust der Lichtstrahlen aufgrund von Transmission durch die Oberfläche weitgehend vermieden ist.

Die Beschichtung der Behälterwand kann mit einem Kunststoffmaterial hinterspritzt sein. Die Beschichtung und die mittels Hinterspritzen hergestellte Schicht aus Kunststoffmaterial, beispielweise einem Polymerwerkstoff, können im Schichtverbund der Behälterwandung eine im Wesentlichen gleiche Schichtdicke oder unterschiedliche Schichtdicken aufweisen. Hierbei kann vorgesehen sein, dass die Beschichtung eine geringere Schichtdicke als die Kunststoffschicht aufweist. Mit dem hinterspritzten Kunststoffmaterial kann eine äußere Behälterwand gebildet sein. Die mittels Hinterspritzen hergestellte Schicht aus dem Kunststoffmaterial bildet hier die äußere Schicht der Behälterwand. Weitere Schichten können für den Aufbau der Behälterwand wahlweise vorgesehen sein.

In einer Ausführungsform ist das die UV-Lichtstrahlen diffus reflektierende Material eingerichtet, UV-Licht in einem Bereich von etwa 200 nm bis etwa 300 nm diffus zu reflektieren.

Das die UV-Lichtstrahlen diffus reflektierende Material kann für die UV-Lichtstrahlen einen Reflexionsgrad von wenigstens etwa 90% aufweisen. Alternativ kann ein Reflexionsgrad von mindestens etwa 95% oder wenigstens etwa 98% ausgebildet sein. In einer Ausführungsform beträgt der Reflexionsgrad für die UV-Lichtstrahlen höchstens etwa 98%.

Das die UV-Lichtstrahlen diffus reflektierende Material ist UV-beständig. Die UV-Beständigkeit kann insbesondere bedeuten, dass sich das die UV-Lichtstrahlen diffus reflektierende Material aufgrund von Bestrahlung mit UV-Licht nicht verfärbt und / oder nicht versprödet. Das die UV-Lichtstrahlen diffus reflektierende Material kann bis zu Temperaturen von etwa 200°C thermisch beständig sein. In einer Ausführungsform kann vorgesehen sein, dass die innere Schicht bis zu einer Temperatur von etwa 260°C thermisch beständig ist.

Es kann insbesondere eine Desinfektion von Wasser oder Trinkwasser vorgesehen sein, um Wasser zu Trinkwasser aufzubereiten oder für vorhandenes Trinkwasser die Wasserqualität mittels Desinfektion zu verbessern.

Es kann vorgesehen sein, dass dem Reaktorraum vorgelagert eine Filtereinrichtung angeordnet ist, in welcher das zu desinfizierende Fluid vor dem Einbringen in dem Reaktorraum gefiltert wird, beispielweise unter Verwendung eines Aktivkohle-Filters. Die Filtereinrichtung kann als Teil der Desinfektionseinrichtung oder dieser vorgelagert sein ausgeführt sein.

Der Behälter kann in Modulbauweise ausgeführt sein, derart, dass das Durchflussrohr aus Behältermodulen zusammengesetzt ist, bei denen an einem Modulkörper mit einem Durchbruch wenigstens eine lichtemittierende UV-Diode angeordnet ist, die im Betrieb in den Durchbruch einstrahlt, und die Durchbrüche der zusammengesetzten Behältermodule können gemeinsam das Durchflussrohr mit der Durchflussstrecke bilden. Bei dieser Ausgestaltung können die Behältermodule mit Hilfe von Verbindungselemente zusammengebaut sein, beispielsweise mittels einer Steckverbindung, um mittels der Durchbrüche der Behältermodule dann die Durchflussstrecke in dem Durchflussrohr bereitzustellen. Die Modulbauweise ermöglicht es, Durchflussrohre unterschiedlicher Länge für verschiedene Anwendungssituationen flexibel bereitzustellen. Der Modulkörper kann einstückig ausgeführt sein, zum Beispiel als Spritzgussbauteil.

In Verbindung mit der Vorrichtung zum Desinfizieren des Fluids gelten die vorangehend in Verbindung mit der Anordnung erläuterten Ausgestaltungen entsprechend.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische perspektivische Darstellung einer Anordnung für eine Vorrichtung zum Desinfizieren eines Fluids mittels UV-Lichts und
- Fig. 2: eine schematische Darstellung einer Vorrichtung zum Desinfizieren eines Fluids.

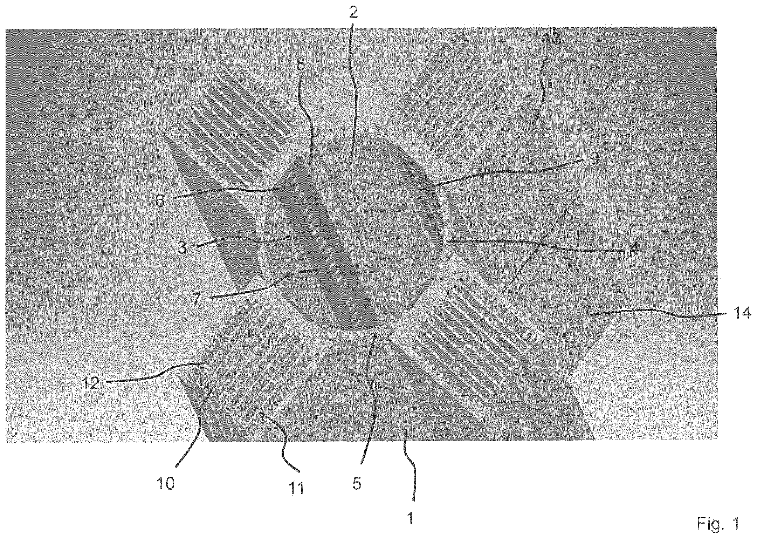

Fig. 1 zeigt eine schematische perspektivische Darstellung einer Anordnung für eine Vorrichtung zum Desinfizieren eines Fluids. Die Anordnung weist einen Behälter 1 auf, bei dem ein Reaktorraum 2 mit einer Durchflussstrecke 3 entlang eines Durchflussrohres 4 gebildet ist. Der Reaktorraum 2 dient dazu, im Betrieb einer Vorrichtung zum Desinfizieren eines Fluids, das zu desinfizierende Fluid aufzunehmen und zum Desinfizieren mit UV-Lichtstrahlen zu bestrahlen. Hierzu sind an einer Behälterwandung 5 lichtemittierende UV-Dioden 6 entlang der Durchflussstrecke 3 angeordnet. Bei dem gezeigten Beispiel sind die lichtemittierenden UV-Dioden 6 in Linienanordnungen von Dioden 7 angeordnet, die sich entlang der Durchflussstrecke 3 durchgehend erstrecken. Die Linienanordnungen von Dioden 7 sind jeweils in einer zugeordneten Ausnehmung 8 der Behälterwandung 5 angeordnet, derart, dass die Durchflussstrecke 3 gegenüber der Umgebung fluiddicht abgedichtet ist.

Mit den lichtemittierenden UV-Dioden 6 ist eine UV-Bestrahlungseinrichtung 9 gebildet. Bei dieser ist den Linienanordnungen von Dioden 7 jeweils eine Kühleinrichtung 10 zugeordnet, die bei der dargestellten Ausführungsform mit einem Kühlkörper 11, welcher Kühlrippen 12 aufweist, gebildet ist, wobei sich die Kühlrippen 12 wie der gesamte Kühlkörper 11 von dem Behälter 1 weg erstrecken. Alternativ oder ergänzend können die Kühleinrichtungen 10 Kühlmitteldurchgänge aufweisen, um diese im Betrieb von einem Kühlmittel durchströmen zu lassen, sodass von den lichtemittierenden UV-Dioden 6 in Betrieb erzeugte Wärmeenergie abgeleitet wird.

Bei der gezeigten Ausführungsform in Fig. 1 sind die lichtemittierenden UV-Dioden 6 um die Durchflussstrecke 3 herum umlaufend angeordnet, insbesondere in paarweise einander gegenüber liegenden Linienanordnung von Dioden. Andere Ausführungen für die Anordnung der lichtemittierenden UV-Dioden 6 um die Durchflussstrecke 3 herum können vorgesehen sein.

Bei der Ausführung in Fig. 1 ist der Behälter 1 aus einem ersten und einem zweiten Behältermodul 13, 14 gebildet, derart, dass Durchgänge des ersten und zweiten Behältermoduls 13, 14 zusammen das Durchflussrohr 4 mit der Durchflussstrecke 3 ausbilden. Die Verbindung zwischen dem ersten und dem zweiten Behältermodul 13, 14 kann lösbar oder nicht lösbar sein. Diese Modulbauweise ermöglicht das Herstellen von Behältern mit unterschiedlich großen Behälterräumen, die sich jeweils entlang des Durchflussrohres 4 erstrecken.

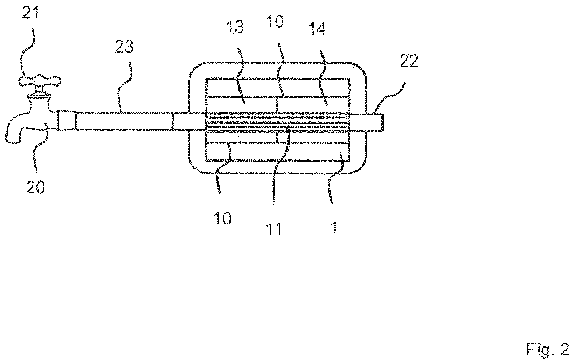

Fig. 2 zeigt eine schematische Darstellung einer Vorrichtung zum Desinfizieren und zum Abgeben eines Fluids, insbesondere von Wasser, beispielsweise Trinkwasser, mit der Anordnung aus Fig. 1. Das desinfizierte Fluid wird an einer Verbrauchsstelle 20, die beispielsweise mit einem Wasserhahn 21 ausgestattet ist, abgegeben. Über einen Zuleitungsanschluss 22 wird das abzugebende und zu desinfizierende Fluid zugeführt, um in den Behälter 1 zum Desinfizieren zu gelangen. Der Auslass des Behälters 1 koppelt an einen Ableitungsanschluss 23, welcher zur Verbrauchsstelle 20 führt.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Anordnung für eine Vorrichtung zum Desinfizieren eines Fluids, mit:
- einem Behälter (1);
- einem Reaktorraum (2), welcher in dem Behälter (1) angeordnet und eingerichtet ist, ein zu desinfizierendes Fluid aufzunehmen, das über einen Einlass des Reaktorraums (2) in diesen gelangen und über einen Auslass des Reaktorraums (2) diesen verlassen kann; und
- einer UV-Bestrahlungseinrichtung (9), die lichtemittierende UV-Dioden (6) aufweist, mit denen UV-Lichtstrahlen zum Desinfizieren des Fluids in den Reaktorraum (2) einstrahlbar sind;
wobei der Reaktorraum (2) in dem Behälter (1) mit einer Durchflussstrecke (3) in einem von einer Behälterwandung (5) umgebenen Durchflussrohr (4) gebildet ist und die lichtemittierenden UV-Dioden (6) an der Behälterwandung (5) im Bereich des Durchflussrohres (4) entlang der Durchflussstrecke (3) verteilt angeordnet sind, um die UV-Lichtstrahlen auf die Durchflussstrecke (3) einzustrahlen.

2. Anordnung nach Anspruch 1, **dadurch gekennzeichnet, dass** zumindest ein Teil der lichtemittierenden UV-Dioden (6) mindestens eine Linienanordnung von Dioden (7) ausbildend an der Behälterwandung (5) angeordnet sind.

3. Anordnung nach Anspruch 2, **dadurch gekennzeichnet, dass** sich die Linienanordnung von Dioden (7) entlang einer Schlitzausnehmung der Behälterwand (5) erstreckt.

4. Anordnung nach mindestens einem der vorangehenden Ansprüche, **dadurch gekennzeichnet, dass** die lichtemittierenden UV-Dioden (6) an der Behälterwandung (5) im Bereich des Durchflussrohres (4) um die Durchflussstrecke (3) herum verteilt angeordnet sind.

5. Anordnung nach mindestens einem der vorangehenden Ansprüche, **dadurch gekennzeichnet, dass** den lichtemittierenden UV-Dioden (6) jeweils eine außerhalb des Reaktorraums (2) angeordnete Kühleinrichtung (10) zugeordnet ist, welche eingerichtet ist, die lichtemittierenden UV-Dioden (6) im Betrieb zu kühlen.

6. Anordnung nach Anspruch 5, **dadurch gekennzeichnet, dass** die Kühleinrichtung (10) sich vom Behälter weg erstreckenden Kühlelementen aufweist.

7. Anordnung nach Anspruch 5 oder 6, **dadurch gekennzeichnet, dass** die Kühleinrichtung (10) einen Kühlmitteldurchgang aufweist, welcher im Betrieb zum Kühlen von einen Kühlmittel durchströmbar ist.

8. Anordnung nach mindestens einem der Ansprüche 5 bis 7, **dadurch gekennzeichnet, dass** Kühleinrichtungen (10) für wenigstens zwei der lichtemittierenden UV-Dioden (6) als gemeinsame Kühleinrichtung ausgebildet sind.

9. Anordnung nach mindestens einem der vorangehenden Ansprüche, **dadurch gekennzeichnet, dass** die Behälterwandung (5) wenigstens oberflächenseitig zumindest abschnittweise aus einem die UV-Lichtstrahlen diffus reflektierenden Material ist.

10. Anordnung nach mindestens einem der vorangehenden Ansprüche, **dadurch gekennzeichnet, dass** der Behälter (1) in Modulbauweise ausgeführt ist, derart, dass das Durchflussrohr (4) aus Behältermodulen (13, 14) zusammengesetzt ist, bei denen an einem Modulkörper mit einem Durchbruch wenigstens eine lichtemittierende UV-Diode (6) angeordnet ist, die im Betrieb in den Durchbruch einstrahlt, und die Durchbrüche der zusammengesetzten Behältermodule (13, 14) gemeinsam das Durchflussrohr (4) mit der Durchflussstrecke (3) bilden.

11. Vorrichtung zum Desinfizieren eines Fluids, mit einer Anordnung nach mindestens einem der vorangehenden Ansprüche.
